(19) Europäisches Patentamt
European Patent Office
Office européen des brevets

(11) EP 0 862 645 B1

(12) EUROPEAN PATENT SPECIFICATION

(45) Date of publication and mention of the grant of the patent:
**05.02.2003 Bulletin 2003/06**

(21) Application number: **96935829.0**

(22) Date of filing: **26.09.1996**

(51) Int Cl.[7]: **C12P 7/42**, C12P 17/14, C07C 57/58, C07D 263/26 // (C12P7/42, C12R1:645), (C12P17/14, C12R1:645)

(86) International application number:
**PCT/US96/14836**

(87) International publication number:
**WO 97/012053 (03.04.1997 Gazette 1997/15)**

(54) **STEREOSELECTIVE MICROBIAL REDUCTION PROCESS**

STEREOSELEKTIVER, MIKROBIELLER REDUKTIONSPROZESS

PROCESSUS DE REDUCTION MICROBIENNE STEREOSELECTIVE

(84) Designated Contracting States:
**AT BE CH DE DK ES FI FR GB GR IE IT LI LU NL PT SE**
Designated Extension States:
**LT LV**

(30) Priority: **27.09.1995 US 4348 P**
**04.01.1996 US 583166**

(43) Date of publication of application:
**09.09.1998 Bulletin 1998/37**

(73) Proprietor: **SCHERING CORPORATION**
**Kenilworth New Jersey 07033 (US)**

(72) Inventors:
- **HOMANN, Michael, J.**
**Clinton, NJ 08809 (US)**
- **PREVITE, Edward**
**North Brunswick, NJ 08902 (US)**

(74) Representative: **Ritter, Stephen David et al**
**Mathys & Squire**
**100 Gray's Inn Road**
**London WC1X 8AL (GB)**

(56) References cited:

| | |
|---|---|
| **EP-A- 0 357 787** | **EP-A- 0 371 568** |
| **EP-A- 0 447 938** | **EP-A- 0 524 595** |
| **WO-A-95/08532** | |

- **TETRAHEDRON (1993), 49(15), 3193-202 CODEN: TETRAB;ISSN: 0040-4020, 1993, XP002022790 BAENS, NICOLE P. ET AL: "Synthesis of 2,5-substituted piperidines: transposition of 1,4-substitution pattern for the analgesic drug R6582"**
- **J. MED. CHEM. (1989), 32(9), 2214-21 CODEN: JMCMAR;ISSN: 0022-2623, 1989, XP002022791 SHIRAISHI, MITSURU ET AL: "Quinones. 4. Novel eicosanoid antagonists: synthesis and pharmacological evaluation"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 862 645 B1

## Description

## BRIEF SUMMARY OF THE INVENTION

The present invention relates to the production of (S)-5-(4-fluorophenyl)-hydroxyvaleric acid (FPHVA) and an S,S diastereomer by microbiological reduction.

EP-A-0 357 787 discloses a procedure for producing an optically active 2-hydroxy acid derivative by microbiological reduction utilising a variety of microorganisms.

EP-A-0 447 938 discloses a process for preparing 2-halogeno-3-hydroxy-3-phenyl-propionic acid ester compounds by permitting an enzyme having the ability of stereoselectively reducing oxo groups to hydroxy groups, to act accordingly on 2-halogeno-3-oxo-s-phenylproprionic acid ester compounds.

The present invention more specifically relates to microbiological reduction of carbonyl groups which comprises cultivating the microorganism *Zygosaccharomyces bailii* American Type Culture Collection (ATCC) 38924, which can be obtained from the American Type Culture Collection, in a medium to which the ketone compound, 4-(4-Fluorobenzoyl)butyric acid (also referred to here as FBBA) can be added so that a compound having a hydroxy group of desired stereochemistry can be formed, accumulated and isolated from said medium. The resulting hydroxy compound, (S)-5-(4-fluorophenyl)hydroxyvaleric acid (FPHVA), is useful as an intermediate in the preparation of 1-(4-fluorophenyl)-3(R) -[3(S)-hydroxy-3-(4-fluorophenyl)propyl)]-4(S)-(4-hydroxyphenyl)-2-azetidinone which is a serum cholesterol lowering agent. More specifically, the hydroxy and acid groups of the hydroxy intermediate, FPHVA, can be protected by protecting group chemistry such as that set forth in Protective Groups in Organic Chemistry. T.W. Greene, John Wiley &Sons (1981). The resulting compound is encompassed by formula III set forth at page 8, line 18 of PCT/US94/10099 published 30 March 1995, which is hereby incorporated by reference. PCT/US94/10099 (WO 95/08532) shows the protected form of FPHVA as being converted to the serum cholesterol lowering agent, mentioned above.

The invention also relates to microbiological reduction of carbonyl groups in FBBA-phenyloxazolidinone conjugate by selected microbes including *Schizosaccharomyces octosporus* ATCC #2479.

## Detailed Description of the Invention

As noted above, this invention relates to a method for performing the following stereospecific reduction using a microorganism.

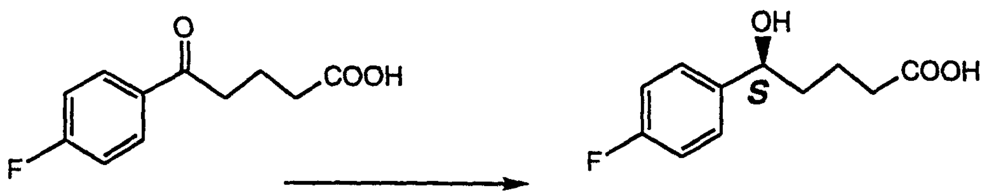

II

I

microbial
reductase

The microbiological reduction is carried out by adding the ketone substrate FBBA (the compound of formula II above), to the culture broth containing the microorganism. The incubation may be conducted at temperatures in the range from between about 20°C and about 40°C, and is preferably conducted at 30°C, while adjusting the initial pH value of the culture medium in the range from between about 4.0 and about 8.0, preferably 5.5.

The initial concentration of compound II in the reaction may vary from between about 1.0g/L and about 20.0 g/L, and is preferably 10.0 g/L.

The duration of the chiral reduction reaction may vary from about 18 to about 96 hours, and is preferably about 48-72 hours.

At the end of the reduction reaction, FPHVA ( the hydroxy compound of formula I), may be extracted using organic solvents such as ethyl acetate, t-butyl methyl ether (TBME), methylene chloride and the like. Adsorption to resins, chromatography, and other physical methods known to the art may also be used to extract compound I.

A large number of microorganisms were investigated to determine whether they reduce the ketone group of compound II. Many such microorganisms failed to provide the desired specificity or productivity. Of those that did provide the desired specificity or productivity for this reduction, *Z. bailii* ATCC No. 38924, afforded the highest yields of compound I with the desired enantiomeric purity, from the highest starting concentration of the ketone, compound

II . This is very desirable, since carrying out this reaction at the highest starting concentration of compound II, affords the most economical means of obtaining the desired alcohol, compound I.
In the examples below are given the following:

Example 1. A means for identifying the stereoselective reduction of compound II by microorganisms including *Z. bailii* ATCC No. 38924.

Example 2. A means for determining that the stereoselective reduction of compound II by cultures including *Z. bailii* ATCC No. 38924, can be carried out at greater concentrations of compound II than in the original identification example.

Example 3. A means for carrying out the stereoselective reduction of compound II by *Z. bailii* ATCC No. 38924, as flask fermentations using greater concentrations of compound II than used in example 2.

Example 4. A means for carrying out the stereoselective reduction of compound II by *Z. bailii* ATCC No. 38924 in flask fermentations, so as to obtain gram quantities of hydroxy compound I.

Example 5. A means for carrying out the stereoselective reduction of compound II by *Z. bailii* ATCC 38924, using a 10-liter fermentor.

The invention also relates to microbiological reduction of carbonyl groups in FBBA-phenyloxazolidinone conjugate (the compound of formula IV) by selected microbes including *Schizosaccharomyces octosporus* ATCC #2479, to obtain the compound of formula III using the appropriate conditions of temperature, initial pH of the starting medium, initial concentration of the compound of formula IV, and duration of the reaction.

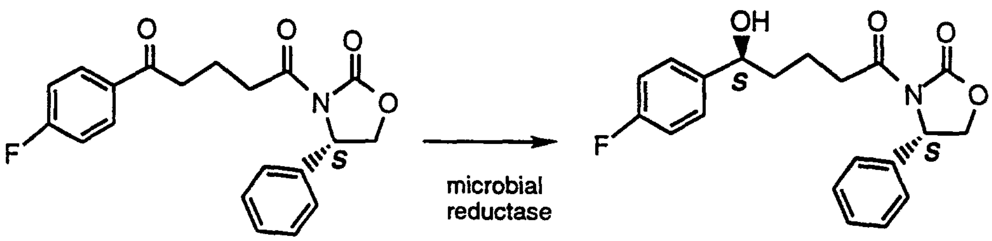


IV III

The resulting compound of formula III may also be converted to cholesterol lowering agents.

## Example 1.

The general method for identifying the stereoselective microbial reduction of 4-(4-Fluorobenzoyl)butyric acid (FBBA, compound II) for use as a synthetic precursor for the production of 1-(4-fluorophenyl)-3(R) -[3(S)-hydroxy-3-(4-fluorophenyl)propyl)]-4(S)-(4-hydroxyphenyl)-2-azetidinone is described below.

Seed cultures of yeast, filamentous fungi, and bacteria were grown in 125 mL flasks containing 25 mL of YPD (1% yeast extract, 2% peptone, 2% dextrose; pH 5.5), SIM6 (3.5% soy flour, 5% white potato dextrose,0.5% cerelose, 2mg/L cobalt chloride, 0.5% calcium carbonate; pH 6.0 ) and NYC (0.8% nutrient broth, 2% yeast extract, 2% cerelose; pH 7.0) media respectively, for 72 hours at 30°C with agitation (175-250 rpm) prior to inoculation (4% v/v) into flask fermentations (25mL YPD/125 mL flask for yeast and filamentous fungi or 25mL NYC /125 mL flask for bacteria) which were incubated at 30°C with agitation (250 rpm). In all fermentations, medium pH was adjusted prior to inoculation but was not controlled during culture propagation and ketone reduction. Ketone reduction was initiated by adding compound II (1 g/L) dissolved in methanol (100 mg/mL) directly to cultures following 24 hours of growth. Samples of fermentation broth extracted with TBME (1:2) following 24-48 hours incubation with substrate were analyzed by reverse-phase HPLC. Cultures demonstrating consistent reduction activity without significant substrate degradation following repeated fermentations using this procedure, were further analyzed by chiral HPLC to determine the configuration of the product alcohol. Cultures capable of reducing compound II at 1 g/L in high enantiomeric excess (ee > 95%) producing compound I the ***S*** enantiomer, are summarized in Table 1.

Table 1.

| **Microorganisms capable of reducing 4-(4- Fluorobenzyol) butyric acid (compound II) at 1 g/L to (S)-5-(4-Fluorophenyl)-5-hydroxy- valeric acid (compound I) with > 95% ee.** | |
|---|---|
| **Genus species** | **ATCC Strain #** |
| *Candida kefyr* | 748<br>14244 |
| *Candida guilliermondii* | 9058<br>20118 |
| *Candida parapsilosis* | 7330<br>16632<br>20008<br>22019<br>34078 |
| *Geotrichum candidum* | 34614 |
| *Hansenula subpelliculosa* | 20281 |
| *Kluyveromyces marxianus* | 8554<br>10022<br>12424 |
| *Saccharomyces cerevisiae* | 26108<br>34081<br>36900 |
| *Saccharomyces uvarum* | 10613<br>32634 |
| *Torulaspora delbrueckii* | 10662 |
| *Torulaspora fermentati* | 20100 |
| *Torulaspora hansenii* | 34022 |
| *Yarrowia lipolytica* | 8661<br>9773 |
| *Zygosaccharomyces bailii* | 36946<br>38924 |

**Example 2.**

The general method for identifying microorganisms including *Z. bailii* ATCC 38924 capable of reducing compound II at concentrations greater than those used in Example 1 is described below.

Seed cultures of yeast were grown in 125 mL flasks containing 25 mL of YPD medium (1% yeast extract, 2% peptone, 2% dextrose; pH 5.5) for 72 hours at 30°C with agitation (250 rpm) prior to inoculation (4% v/v) into flask fermentations (25 mL YPD/125 mL flask) incubated at 30°C with agitation (250 rpm). In all fermentations, medium pH was adjusted prior to inoculation but was not controlled during culture propagation and ketone reduction. Ketone reduction was initiated by adding compound II (1-4 g/L) dissolved in methanol (100 mg/mL) directly to cultures at inoculation, following 24 hours of growth or both as indicated in Table 2. Samples of fermentation broth extracted with TBME (1:2) following 24-48 hours incubation with compound II were analyzed by HPLC to assess the yield of compound I as summarized in Table 2.

Table 2.

| **Cultures capable of reducing Compound II at 1-4 g/L in flask fermentations.** | | |
|---|---|---|
| **Genus species ATCC #** | **Condition** | **% Yield, Compound I** |
| | 1 g/L at log 24 | 98 |

Table 2. (continued)

| Cultures capable of reducing Compound II at 1-4 g/L in flask fermentations. | | |
|---|---|---|
| **Genus species ATCC #** | **Condition** | **% Yield, Compound I** |
| *Candida kefyr 748* | 2 g/L at log 24<br>4 g/l at log 24 | 70<br>34 |
| *Candida kefyr 14244* | 1 g/Lat log 24<br>2 g/L at log 24<br>4 g/L at log 24 | 95<br>80<br>28 |
| *Candida guilliermondii 9058* | 1 g/L at log 24<br>2 g/L at log24<br>4 g/L at log 24 | 90<br>9<br>0 |
| *Candida parapsilosis 7330* | 1 g/L at log 24<br>1 g/L at logs 0+24<br>4 g/L at log 24 | 100<br>88<br>6 |
| *Candida parapsilosis 16632* | 1 g/L at log 24<br>1 g/L at logs 0+24<br>4 g/L at log 24 | 100<br>100<br>18 |
| *Candida parapsilosis 22019* | 1 g/L at log 24<br>1 g/L at logs 0+24<br>4 g/L at log 24 | 100<br>99<br>15 |
| *Kluyveromyces marxianus 8554* | 1 g/L at log 24<br>2 g/L at log 24<br>4 g/L at log 24 | 86<br>93<br>32 |
| *Saccharomyces cerevisiae 26108* | 1 g/L at log 24<br>1 g/L at logs 0+24<br>4 g/L at log 24 | 98<br>42<br>13 |
| *Saccharomyces cerevisiae 34081* | 1 g/L at log 24<br>1 g/L at logs<br>0+24<br>4 g/Lat log 24 | 92<br>83<br>34 |
| *Saccharomyces cerevisiae 36900* | 1 g/L at log 24<br>1 g/Lat logs 0+24<br>4 g/L at log 24 | 95<br>66<br>25 |
| *Saccharomyces uvarum 32634* | 1 g/L at log 24<br>1 g/L at logs 0+24<br>4 g/L at log 24 | 94<br>47<br>6 |
| *Torulaspora delbrueckii 10622* | 1 g/L at log 24<br>2 g/L at log 24<br>4 g/L at log 24 | 68<br>42<br>10 |
| *Torulaspora* | 1 g/L at log 24<br>2 g/L at log 24 | 95<br>30 |

Table 2. (continued)

| Cultures capable of reducing Compound II at 1-4 g/L in flask fermentations. | | |
|---|---|---|
| **Genus species ATCC #** | **Condition** | **% Yield, Compound I** |
| *fermentati 20100* | 4 g/L at log 24 | 6 |
| *Zygosaccharomyces bailii 36946* | 4 g/L at log 24 | 84 |
| *Zygosaccharomyces bailii 38924* | 4 g/L at log 24 | 100 |

**Example 3.**

The general method for investigating the fermentation parameters for the reduction of compound II by *Z. bailii* ATCC 38924 in flask fermentations is described below. Seed cultures of *Z. bailii* ATCC 38924 were grown in 125 mL or 300 mL flasks containing 25mL or 100 mL of YPD (1% yeast extract, 2% peptone, 2% dextrose; pH 5.5) or TNC (1% Tastone 154, 2% NZ amine, 3% cerelose; pH 5.5) for 24-72 hours at 30°C with agitation (250 rpm) prior to inoculation (2-8 % v/v) into flask fermentations (25mL-100 mL YPD or TNC /125-300 mL flask) which were incubated at 30°C with agitation (250 rpm) where indicated in Table 3. In all conditions, medium pH was adjusted prior to inoculation but was not controlled during culture propagation and ketone reduction. Ketone reduction was initiated by adding compound II (4-10 g/L) dissolved in methanol (MeOH; 100 mg/mL) or dimethylsulfoxide (DMSO; 500 mg/mL with slight heat) directly to cultures following 24 hours of growth. Samples of fermentation broth were taken following 24-72 hours of incubation with compound II and extracted with TBME (1:4). Extracts were diluted with ethanol (1:1) prior to analysis by reverse-phase HPLC to determine the yield of compound I as summarized in Table 3.

**Example 4.**

Gram quantities of compound I derived from the stereoselective reduction of compound II were prepared using *Z. bailii* ATCC 38924 in flask fermentations employing conditions affording the highest yield and selectivity as summarized in Table 3 parameter set 27. Specifically, seed cultures of *Z. bailii* ATCC 38924 were grown in 300 mL flasks containing 100 mL of TNC medium (1% Tastone 154, 2% NZ amine, 3% cerelose; pH 5.5) for 72 hours at 30°C with agitation (250 rpm). Medium pH was adjusted prior to inoculation but was not controlled during culture propagation or subsequently during ketone reduction. Seed inoculum (4% v/v) was transfered into fermentation flasks (300 mL) containing 100 mL of TNC medium and propagated at 30°C with agitation (250 rpm) for 24 hours prior to the addition of 10 g/L of compound II dissolved in dimethylsulfoxide (DMSO; 500 mg/mL with slight heat). Following 24 hours of incubation with compound II, additional cerelose (3% w/v) was added to the fermentation. Samples of fermentation broth taken during incubation with compound II, were extracted with TBME (1:4), diluted with ethanol (1:1) and analyzed by reverse-phase HPLC. Reduction of compound II to compound I was shown to be complete following 72 hours of incubation.

Compound I was recovered from the fermentation broth (5 X 100 mL) by removing cells by centrifugation and extracting the supematant with TBME (2X 250 mL) and ethyl acetate(1X200 mL). The pooled extract was washed with distilled water and saturated sodium chloride solution (2X 250 mL). Anhydrous magnesium sulfate was added to the washed solvent extract to adsorb residual water and removed by filtration. The filtrate was concentrated by evaporation and subjected to purification by silica gel chromatography (2 x 0.127 - 0.254 m 2 x 5-10 inch column bed). Material was eluted from the column with a solution of methylene chloride:ethyl acetate (60:40). Peak fractions containing only compound I were identified by thin layer chromatography, pooled and concentrated by evaporation. A total of 3.43 g of compound I was isolated from a 5 g bioconversion. Samples of this material were confirmed to be the desired product compound I, by reverse phase and chiral HPLC, NMR, mass spectrum and elemental analyses.

**Example 5.**

The general method for the bioconversion of compound II to compound I using *Z. bailii* ATCC 38924 in 10 liter fermentors is described below.

First stage seed cultures were grown in TNC medium (1% Tastone 154, 2% NZ amine, 3% cerelose; pH 5.5) using 100mL/300 mL flasks for 24 hours at 30° C with agitation (250 rpm) prior to serial transfer (5%) into second stage seed fermentations employing TNC medium (500 mL/ 2 L flask) incubated at 30° C with agitation (200 rpm). Following 72 hours of growth, second stage seed cultures were used to inoculate (5 % v/v) fermentors containing 5-10 liters of BTX-1-XO2 medium (1% Tastone 154, 2% NZ amine, 12% cerelose, 0.5 mL SAG-471 antifoam/L medium; pH 5.5). The culture was propagated in the fermentor at 30° C, with agitation (impellor speed; 600-1000 rpm) and aeration

(airflow; 3-10 Lpm) under pressure (34500 Pa) 5 psi. Dissolved oxygen was maintained at or above 30% by adjusting agitation speed and aeration rates. Culture growth was monitored by analyzing off gas for carbon dioxide and medium pH was adjusted prior to inoculation but was not controlled during culture propagation and ketone reduction. Ketone reduction was initiated by adding compound II (10 g/L) dissolved in dimethyl sulfoxide (400 mg/mL) directly to cultures when the off gas attained an initial concentration of 3.9%. Cerelose (3% w/v) was added to the ongoing fermentation when the off gas attained both an initial concentration of 2.5 %, and a maximum carbon dioxide evolution. Additional 6% cerelose was added following maximum carbon dioxide evolution when the off gas contained 2% carbon dioxide. Samples of fermentation broth were taken following 24-72 hours of incubation with compound II and extracted with TBME (1:4). Extracts were diluted with ethanol (1:1) prior to analysis by reverse-phase HPLC. Approximately 80-90% of compound II was reduced to compound I following 48-72 hours of incubation.

Table 3.

| Reduction of Compound II (4-10 g/L) by *Z. bailii* ATCC 38924 in flask fermentations. | | | | | | | |
|---|---|---|---|---|---|---|---|
| Parameter Set # | Seed Conditions | Seed Age (hour) | Transfer Volume (%v/v) | Bioconversion Conditions (media vol / flask vol.) | Compound II (g/L) | Solvent | % Yield Compound I |
| 1 | YPD | 24 | 2 | YPD | 4 | MeOH | 55 |
| 1a | 25 mL/ 125mL | | | 25 mL 125mL | 5 | | 37 |
| 2 | 250 rpm, 30 C | | 4 | 250 rpm, 30 C | 4 | | 41 |
| 2a | | | | | 5 | | 34 |
| 3 | | | 8 | | 4 | | 36 |
| 3a | | | | | 5 | | 33 |
| 4 | | 48 | 2 | | 4 | | 48 |
| 4a | | | | | 5 | | 43 |
| 5 | | | 4 | | 4 | | 47 |
| 5a | | | | | 5 | | 33 |
| 6 | | | 8 | | 4 | | 32 |
| 6a | | | | | 5 | | 28 |
| 7 | | 72 | 2 | | 4 | | 72 |
| 7a | | | | | 5 | | 48 |
| 8 | | | 4 | | 4 | | 57 |
| 8a | | | | | 5 | | 42 |
| 9 | | | 8 | | 4 | | 56 |
| 9a | | | | | 5 | | 39 |
| 10 | YPD | 24 | 4 | YPD | 4 | MeOH | 95 |
| 10a | 25 mL/ 125mL | | | 50 mL/ 300 mL | 5 | | 46 |
| 11 | 250 rpm, 30 C | 48 | | 250 rpm, 30 C | 4 | | 48 |
| 11a | | | | | 5 | | 40 |
| 12 | | 72 | | | 4 | | 72 |
| 12a | | | | | 5 | | 57 |
| 13 | YPD | 24 | 4 | YPD | 4 | MeOH | 33 |
| 13a | 25 mL/ 125mL | | | 30 mL/ 300 mL | 5 | | 22 |
| 14 | 250 rpm, 30 C | 48 | | 250 rpm, 30 C | 4 | | 36 |
| 14a | | | | | 5 | | 26 |
| 15 | | 72 | | | 4 | | 42 |

Table 3. (continued)

| Reduction of Compound II (4-10 g/L) by *Z. bailii* ATCC 38924 in flask fermentations. | | | | | | | |
|---|---|---|---|---|---|---|---|
| Parameter Set # | Seed Conditions | Seed Age (hour) | Transfer Volume (%v/v) | Bioconversion Conditions (media vol / flask vol.) | Compound II (g/L) | Solvent | % Yield Compound I |
| 15a | | | | | 5 | | 31 |
| 16 | YPD | 24 | 4 | YPD | 4 | MeOH | 11 |
| 16a | 25 mL 125mL | | | 50 mL/ 300 mL | 5 | | 11 |
| 17 | 250 rpm, 30 C | 48 | | Baffled flask | 4 | | 10 |
| 17a | | | | 250 rpm, 30 C | 5 | | 10 |
| 18 | | 72 | | | 4 | | 19 |
| 18a | | | | | 5 | | 13 |
| 19 | YPD | 24 | 4 | YPD | 4 | MeOH | 100 |
| 19a | 25 mL 125mL | | | 100 mL/ 300mL | 5 | | 91 |
| 20 | 250 rpm, 30 C | 48 | | 250 rpm, 30 C | 4 | | 100 |
| 20a | | | | | 5 | | 84 |
| 21 | | 72 | | | 4 | | 121 |
| 21a | | | | | 5 | | 100 |
| 22a | TNC | 72 | 2 | TNC | 4 | MeOH | 99 |
| 22b | 25 mL 125mL | | | 100 mL/ 300mL | 5 | | 100 |
| 22c | 250 rpm, 30 C | | | 250 rpm, 30 C | 6 | | 94 |
| 22d | | | | | 8 | | 39 |
| 23a | TNC | 72 | 4 | TNC | 4 | MeOH | 99 |
| 23b | 25 mL/ 125mL | | | 100 mL/ 300mL | 5 | | 90 |
| 23c | 250 rpm, 30 C | | | 250 rpm, 30 C | 6 | | 88 |
| 23d | | | | | 8 | | 62 |
| 24 | TNC | 72 | 4 | TNC | 6 | MeOH | 85 |
| 24a | 100mL/ 300mL 250 rpm, 30 C | | | 100 mL/ 300 mL 250 rpm, 30 C | 8 | | 50 |
| 25 | TNC | 72 | 4 | TNC | 8 | DMSO | 78 |
| 25a | 100 mL/ 300mL 250 rpm, 30 C | | | 100 mL/300 mL 250 rpm, 30 C | 10 | | 76 |
| 26 | TNC 100 mL/ 300mL | 72 | 4 | TN2C (contains 6% cerelose) | 10 | DMSO | 82-90 |

Table 3. (continued)

| Reduction of Compound II (4-10 g/L) by *Z. bailii* ATCC 38924 in flask fermentations. | | | | | | | |
|---|---|---|---|---|---|---|---|
| Parameter Set # | Seed Conditions | Seed Age (hour) | Transfer Volume (%v/v) | Bioconversion Conditions (media vol / flask vol.) | Compound II (g/L) | Solvent | % Yield Compound I |
| | 250 rpm, 30 C | | | 100 mL/ 300 mL 250 rpm, 30 C | | | |
| 27 | TNC 100 mL 300mL 250 rpm, 30 C | 72 | 4 | TNC + 3% cerelose 24h post substrate addition<br><br>100 mL/300 mL 250 rpm, 30 C | 10 | DMSO | 100 |

**Example 6.**

**Reduction of FBBA-phenyloxazolidinone conjugate by selected microbes including *Schizosaccharomyces octosporus* ATCC #2479.**

The general method for identifying the stereoselective microbial reduction of the condensation product compound IV, formed from 4-(4-Fluorobenzoyl)butyric acid (FBBA) and phenyloxazolidinone, to compound III for use as a synthetic precursor for the production of 1-(4-fluorophenyl)-3(R) -[3(S)-hydroxy-3-(4-fluoro-phenyl)propyl)]-4(S)-(4-hydroxyphenyl)-2-azetidinone is described below.

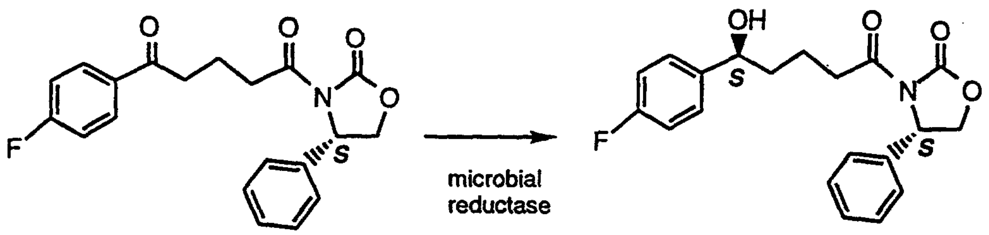


IV III

Seed cultures of yeast, filamentous fungi, and bacteria were grown in 125 mL flasks containing 25 mL of YPD (1% yeast extract, 2% peptone, 2% dextrose; pH 5.5), SIM6 (3.5% soy flour, 5% white potato dextrose, 0.5% cerelose, 2mg/L cobalt chloride , 0.5% calcium carbonate; pH 6.0) and NYC (0.8% nutrient broth, 2% yeast extract, 2% cerelose; pH 7.0) media respectively, for 72 hours at 30°C with agitation (175-250 rpm) prior to inoculation (4 % v/v) into flask fermentations. In all fermentations, medium pH was adjusted prior to inoculation but was not controlled during culture propagation and ketone reduction. Ketone reduction was initiated by adding compound IV (1 g/L) dissolved in methanol (100 mg/mL) directly to cultures following 24 hours of growth in (25mL YPD/125 mL flask for yeast and filamentous fungi or 25mL NYC /125 mL flask for bacteria) incubated at 30° C with agitation (250 rpm). Synthesis and enantiomeric excess of compound III were determined by chiral HPLC using samples of fermentation broth extracted with TBME (1: 2) following 24-48 hours incubation with compound IV. Cultures capable of reducing compound IV yielding compound III are summarized in Table 4.

*Schizosaccharomyces octosporus* ATCC 2479 afforded the highest yield of compound III with the desired enantiomeric purity from 1 g/L of compound IV.

Table 4.

| Microbes capable of reducing compound IV (FBBA- phenyloxazolidinone conjugate) to compound III (S,S diastereomer) with ≥ 95% de. | |
|---|---|
| **Genus species** | **ATCC strain #** |
| *Rhodococcus erythropolis* | 4277 |
| *Rhodococcus globerulus* | 21505 |
| *Rhodococcus rhodochrous* | 9356 |
| *Mucor mucedo* | 20094 |
| *Mucor racemosus* | 7924 |
| *Mucor circinelloides* | 1207a<br>7941<br>56641 |
| *Rhizopus oryzae* | 11145<br>24563 |
| *Aspergillus carneus* | 16798<br>20231 |
| *Aspergillus niveus* | 12276 |
| *Schizosaccharomyces octosporus* | 2479<br>4206 |

Multiple flask bioconversions were conducted employing *S. octosporus* ATCC 2479 to generate material for chemical characterization. Seed cultures were grown in YPD medium (25 mL/125 mL flask) for 72 hours at 30° C with agitation prior to inoculation (4 % v/v) into flask fermentations (20 X 100 mL/300mL). Medium pH was adjusted prior to inoculation but was not controlled during culture propagation and ketone reduction. Compound IV (1 g/L dissolved in methanol at 100 mg/mL) was added to flask fermentations after 24 hours of growth at 30°C with agitation (250 rpm). Compound IV was reduced to compound III in 20-40% yield. Degradative hydrolysis of compound IV was also observed. The fermentation broth was pooled, centrifuged to remove cells and the supernatant extracted with ethyl acetate (1: 0.5). The extract was washed twice with an equal volume of salt solution (6M sodium chloride) followed by deionized distilled water. Anhydrous magnesium sulfate was added to the ethyl acetate extract to remove residual water, the extract was filtered and the filtrate concentrated by evaporation. Extract concentrate was subjected to purification by silica gel chromatography (0·025 x 0·305m 1 - 12 inch column bed). Material was eluted from the column with a solution of methylene chloride:ethyl acetate: acetic acid (90:10:0.5). Peak fractions containing products were pooled and concentrated by evaporation, followed by separation on preparative thin layer chromatography plates employing a solution of ethyl acetate: hexane: acetic acid (60:40:0.5). Two products were isolated from the fermentation broth using this procedure and shown to be the desired reduced alcohol conjugate compound III and the phenyloxazolidinone reagent used to synthesize compound IV, as determined by HPLC and NMR analyses.

## Claims

1. A stereoselective reduction of a compound of formula II to compound of formula I

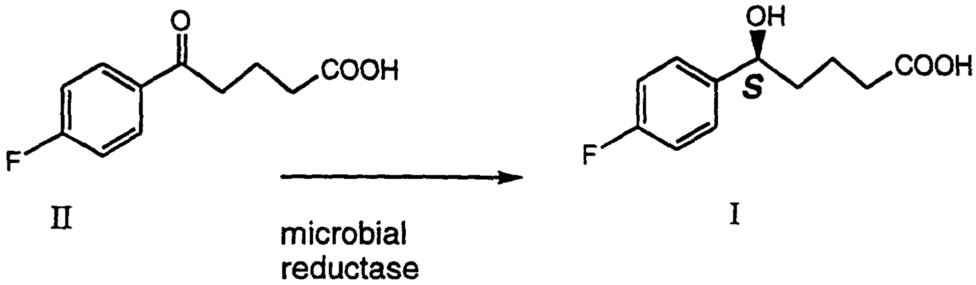


II I

which comprises adding a compound of formula II to a culture broth of *Zygosaccharomyces bailii* ATCC 38924, incubating the resulting mixture, and isolating a hydroxy compound of formula I.

**2.** A stereoselective reduction of a compound of formula IV to compound of formula III

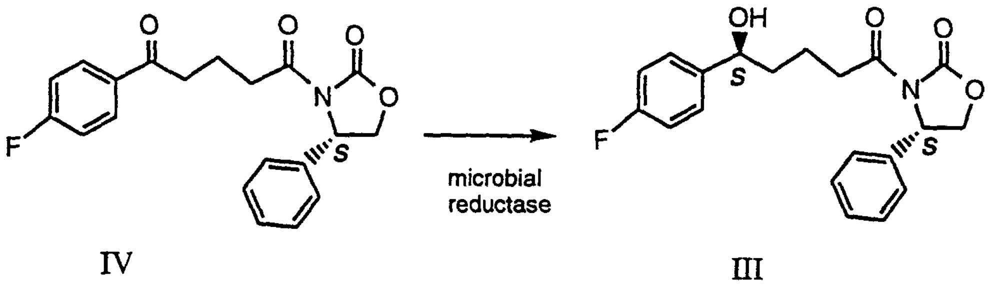


IV III

which comprises adding a compound of formula IV to a culture broth of *Schizosaccharomyces octosporus* ATCC 2479, incubating the resulting mixture, and isolating a hydroxy compound of formula III.

**Patentansprüche**

**1.** Stereoselektive Reduktion einer Verbindung der Formel II zu einer Verbindung der Formel I

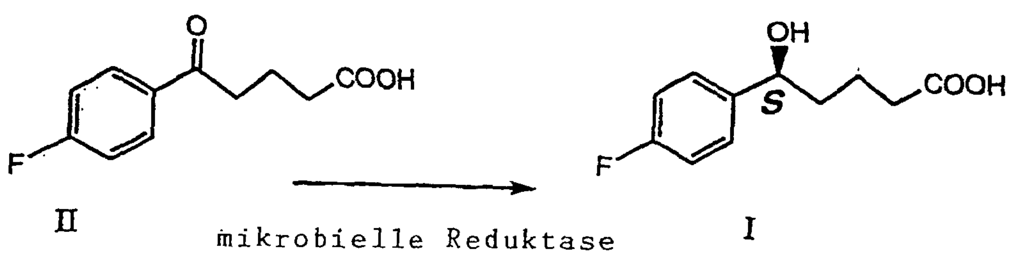


II I

Schritte umfassend, bei denen man eine Verbindung der Formel II zu einer Kulturbrühe von *Zygosaccharomyces bailii* ATCC 38924 gibt, die so erhaltene Mischung inkubiert und eine Hydroxyverbindung der Formel I isoliert.

**2.** Stereoselektive Reduktion einer Verbindung der Formel IV zu einer Verbindung der Formel III

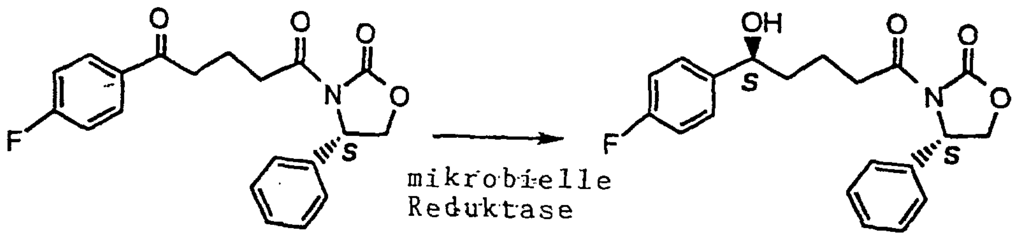


IV

III

Schritte umfassend, bei denen man eine Verbindung der Formel IV zu einer Kulturbrühe von *Schizosaccharomyces octosporus* ATCC 2479 gibt, die erhaltene Mischung inkubiert und eine Hydroxyverbindung der Formel III isoliert.

## Revendications

**1.** Réduction stéréosélective du composé de formule II en le composé de formule I :

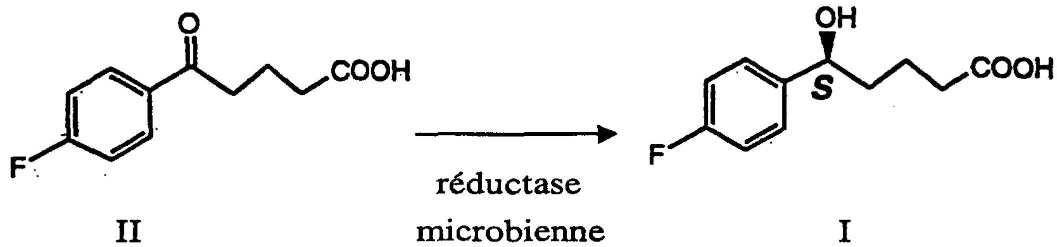


II

I

qui comporte le fait d'ajouter du composé de formule II à un bouillon de culture de *Zygosaccharomyces bailli* ATCC 38924, le fait de faire incuber le mélange résultant, et le fait d'isoler le composé hydroxylé de formule I.

**2.** Réduction stéréosélective du composé de formule IV en le composé de formule III :

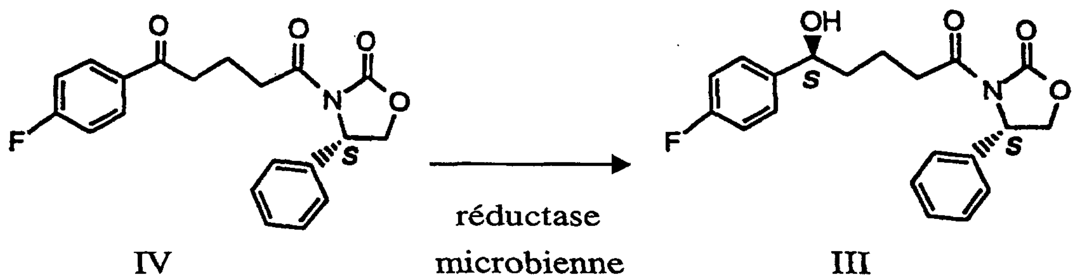


IV

III

qui comporte le fait d'ajouter du composé de formule IV à un bouillon de culture de *Schizosaccharomyces octosporus* ATCC 2479, le fait de faire incuber le mélange résultant, et le fait d'isoler le composé hydroxylé de formule III.